# EUROPÄISCHE PATENTANMELDUNG

(11) **EP 1 958 597 A1**
(43) Veröffentlichungstag der Anmeldung: **20.08.2008**
(21) Anmeldenummer: 07003350.1
(22) Anmeldetag: 16.02.2007
(51) Int. Cl.: A61F 2/90

(54) **Rohrförmige Stützprothese mit Herzklappe insbesondere für Aortenklappenersatz**

(71) Anmelder: Universität Zürich, 8006 Zürich (CH)
(72) Erfinder: Die Erfindernennung liegt noch nicht vor
(74) Vertreter: Grünecker, Kinkeldey, Stockmair & Schwanhäusser Anwaltssozietät

(57) **Zusammenfassung**

Die Erfindung betrifft eine rohrförmige Stützprothese aufweisend zwei auf die Stützprothesenlängsachse bezogene endständige Bereiche und einen zwischen den beiden endständigen Bereichen angeordneten Mittelbereich, wobei jeder endständige Bereich ein Maschengerüst aus zumindest zwei miteinander über Verbindungsglieder verbundenen, punktsymmetrisch um die Stützprothesenlängsachse angeordneten Gerüstringen aufweist, wobei der Mittelbereich durch langgezogene Verbindungsglieder gebildet ist, die mit den jeweils dem Mittelpunkt der Stützprothesenlängsachse am nächsten angeordneten Gerüstringen verbunden sind, und wobei eine mittels Tissue-Engineering hergestellte Aortenklappe im Mittelbereich befestigt und/oder integriert ist.

## Beschreibung

Die Erfindung betrifft Stützprothesen mit Herzklappen. Insbesondere betrifft die Erfindung Stützprothesen mit Herzklappen insbesondere für Aortenklappenersatz.

In der Regel werden Klappenprothesen als integrierter Teil von Gefäßstützprothesen in den Körper eingebracht. Solche Stützprothesen sind in der Regel rohrförmige Gitterstrukturen, die man auch als "Stents" bezeichnet. Stützprothesen mit integriertem Klappenersatz sind im Stand der Technik bekannt. Solche Klappen werden aus der Kuh, aus dem Schwein sowie aus dem Pferd gewonnen. Auch sind Stützprothesen mit tissue-engineerten ("TE") Klappen bekannt (Stock et al. (2006). J. Thorac. Cardiovasc. Surg. 131, 1323-30) Im Allgemeinen bietet Tissue-Engineering gegenüber einem Xenograft den Vorteil, dass Zellen beliebiger Herkunft (z.B. homologe oder autologe menschliche Zellen) in die Struktur einer existierenden, dezellularisierten nicht menschlichen Klappe integriert werden können (vgl. EP 1499366).

Das Implantieren solcher Stützprothesen mit TE-Klappen hat sich aber in der Praxis als äußerst schwierig gestaltet, da TE-Klappen in der Regel sehr empfindlich sind. So können solche Klappen, wenn sie innerhalb einer Stützprothese, die beispielsweise aus Metall besteht, in den Körper eingeführt werden, von der metallenen Struktur dieser Stützprothese eingerissen oder zerquetscht werden und/oder sich an Teilen innerhalb der Stützprothese sich verfangen. Dies führt zu einer unerwünschten Beschädigung der im Stent angeordneten Klappe. Bei einer TE-Klappe, die in dieser Art und Weise beschädigt worden ist, kann es zu Degeneration und/oder Fehlfunktion der Klappe kommen.

Im Stand der Technik hat man dieses Problem dadurch gelöst, dass die Innenseite eines eine Klappe enthaltenen Stents mit Xenograftgewebe ausgekleidet wird. So hat man beispielsweise eine Mukosaschicht aus dem Dünndarm des Schweins an die Innenseite eines Metallstents genäht. Anschliessend wurde eine Pulmonarklappe danach mit dieser Mukosaschicht verbunden. Die Mukosaschicht diente als eine Art Abschirmung gegen die die Klappe potenziell beschädigende Metallstruktur des Stents (Stock et al. (2006). J. Thorac. Cardiovasc. Surg. 131, 1323-30). Man hat festgestellt, dass die Herzklappe bei einem solchen, an seiner Innenseite mit Gewebe ausgekleideten Stent weniger stark beschädigt wurde als Klappen bei Stents, die überhaupt keine Gewebeschicht oder eine Gewebeschicht nur au-ßenseitig aufwiesen.

Trotzdem sind solche an ihren Innenseiten mit Gewebeschichten ausgekleideten Prothesen mit erheblichen Nachteilen verbunden. Zuerst wird die Vorbereitung auf das Implantieren durch die Notwendigkeit, eine zusätzliche Schicht an der Innenseite eines Stents anzubringen, verkompliziert. Zweitens bringt die Verwendung von Gewebeschichten nicht menschlicher Herkunft erhebliche Risiken hinsichtlich einer Infektion und/oder einer Transplantatabstoßung im menschlichen Patienten mit sich. Andererseits ließe sich schwerlich aus jedem Patienten, der eine Klappenprothese benötigt, für ein Auskleiden des einzuführenden Stents ausreichende Mengen an autologer Mukosaschicht isolieren.

Unabhängig von diesen Nachteilen bei bekannten Stents, die eine Ersatzklappe enthalten, bestehen im speziellen Zusammenhang der Aortenklappenprothesen erhebliche Schwierigkeiten. Diese Schwierigkeiten ergeben sich teilweise aus der generellen Empfindlichkeit von Klappenprothesen, wie oben erläutert, aber auch aus den anatomischen Besonderheiten speziell im Aortenbereich. Aufgrund des hohen Blutdrucks im Aortenbereich wird das Aortengewebe außerordentlichen physischen Belastungen ausgesetzt. Um diesen Belastungen standzuhalten, und dabei noch seine Stützfunktion zu erfüllen, muß ein für den Aortenbereich vorgesehener Stent daher robust sein, wobei eine Unnachgiebigkeit in der Stentstruktur eine hohe Beschädigungsgefahr für die sich darin befindende Klappe bedeutet. Ferner birgt die Einbringung eines sehr robusten Stents im Aortenbereich die Gefahr in sich, dass andere lebenswichtige Gefäße, wie zum Beispiel die beidseitig von der Aorta abführenden Koronararterien Ostium dextra und Ostium sinistra, in ihrer Funktion beeinträchtigt werden.

Somit ist es eine Aufgabe der vorliegenden Erfindung, einen verbesserten Stent mit Klappenersatz bereitzustellen, der das Implantieren einer empfindlichen TE-Klappe in stark beanspruchte Bereichen wie in die Aorta ermöglicht, wobei die oben erwähnten Nachteile vermieden werden.

Erfindungsgemäß wird diese Aufgabe durch das Bereitstellen einer rohrförmigen Stützprothese gelöst, die auf die Stützprothesenlängsachse bezogen zwei endständige Bereiche und einen zwischen den zwei endständigen Bereichen angeordneten Mittelbereich aufweist, wobei jeder endständige Bereich ein Maschengerüst aus zumindest zwei miteinander über Verbindungsglieder verbundenen, punktsymmetrisch um die Stützprothesenlängsachse angeordneten Gerüstringen aufweist, wobei der Mittelbereich durch langgezogene Verbindungsglieder gebildet ist, die mit den jeweils dem Mittelpunkt der Stützprothesenlängsachse am nächsten angeordneten Gerüstringen verbunden sind, und wobei eine mittels Tissue-Engineering hergestellte Aortenklappe im Mittelbereich befestigt/und oder integriert ist.

Ein solcher Stützprothesenaufbau bringt einige überraschende Vorteile mit sich.

Erstens ist die Aortenklappe in dem mittleren Bereich angeordnet, d. h., sie ist nicht primär mit dem proximalen oder dem distalen Maschengerüst verbunden. Bei der erfindungsgemä-ßen Stützprothese stellen die zwei endständigen Maschengerüste die Bereiche der höchsten Robustheit, d.h. Starrheit dar, wohingegen der durch die langgezogenen Verbindungsglieder gebildete Mittelbereich im Vergleich weniger starr ist. Durch die Befestigung der empfindlichen TE-Aortenklappe an diesem vergleichsweise weniger starren Mittelbereich, werden potentiell beschädigende Wechselwirkungen zwischen der Aortenklappe und den endständigen Maschengerüsten weitgehend vermieden.

Zweitens kann durch die Festlegung der Aortenklappenposition im Mittelbereich der Stützprothese die typischerweise durch das Aufdehnen der Stützprothese mittels eines Ballonkatheters während des Implantiervorgangs verursachte Beschädigung der Klappe vermieden werden. So kann die erfindungsgemäße Stützprothese mit Hilfe eines zweiteiligen Ballonkatheters an den richtigen Ort im Körper gebracht werden, wobei der eine Teil des Ballonkatheters das Maschengerüst im ersten endständigen Bereich der Stützprothese aufbläht und der zweite Teil des Ballonkatheters das Maschengerüst im zweiten endständigen Bereich der Stützprothese aufbläht. Im Mittelbereich der Stützprothese, d. h. in dem Bereich, in dem sich die TE-Aortenklappe befindet, findet während des Einführens und Expandierens der endständigen Bereiche der Stützprothese keine Ballon-Expansion statt, d.h. der Ballon ist in seinem Mittelbereich nicht oder nur schwer expandierbar, so dass die Aortenklappe nicht gegen die umliegende Prothesenstruktur gedrückt wird. Auf diese Weise ermöglicht die erfindungsgemäße Stützprothese eine Verankerung in der Aorta und/oder im linksventrikulären Ausflusstrakt, ohne dass die empfindliche, sich innerhalb der Stützprothese befindende TE-Aortenklappe beschädigt wird.

Drittens wird durch die lang gezogenen Verbindungsglieder im Mittelbereich der Stützprothese erreicht, dass dieser Mittelbereich gegenüber den beiden Maschengerüst aufweisenden endständigen Bereichen der Stützprothese erheblich lockerer ist. So wird ein zusätzliches Problem, das üblicherweise mit dem Aortenklappenersatz auftritt, vermieden, nämlich eine Beeinträchtigung der von der Aorta abzweigenden Koronararterien Ostium dextra und Ostium sinistra. Die erfindungsgemäße Stützprothese kann daher so in den Patientenkörper eingebracht werden, dass der lockere Mittelbereich der Stützprothese in derselben Höhe wie diese beiden abzweigenden Koronararterien liegt, wodurch eine Beeinträchtigung oder sogar eine Okklusion dieser lebenswichtigen Arterien vorteilhaft ausgeschlossen wird.

Gemäß einer Ausführungsform der vorliegenden Erfindung weist zumindest ein Gerüstring des Maschengerüstes n periodische, längs der Stützprothesenlängsachse sich erstreckende, Hoch- und Tiefpunkte bildende Verformungen auf, die eine sich auf die Stützprothesenlängsachse bezogene Amplitude A aufweisen, wobei n = 16-70, bevorzugt 20-56, besonders bevorzugt 24-42. Hierbei ist A als die maximale Auslenkung einer jeweiligen Verformung aus ihrer Mittellage, also aus einer für jeden Gerüstring definierten Umfangsmittellinie M (wobei M eine Fläche einschließt, die die Stützprothesenlängsachse senkrecht schneidet) zu verstehen, und beträgt 0,25-8 mm, bevorzugt 0,75-4 mm, besonders bevorzugt 1-2 mm. Diese Verformungen, die sinusförmig, rechteckig, sägezahnförmig, dreieckig oder mäanderförmig, vorzugsweise sinusförmig, ausgestaltet sein können, verleihen den endständigen Bereichen der Stützprothese ihre für das Einführen mittels Katheter notwendige Faltbarkeit und Elastizität, d. h. sie ermöglichen, dass die endständigen Bereiche der Stützprothese vor dem Einführen in einen Patienten auf den benötigten Durchmesser reduziert werden können, ohne sich in ihrer Gesamtlänge wesentlich zu verkürzen.

Gemäß einer weiteren Ausführungsform weist jedes endständige Maschengerüst 2-8, vorzugsweise 2-6, besonders bevorzugt 2-4 Gerüstringe auf.

Gemäß einer weiteren Ausführungsform der vorliegenden Erfindung ist die Anzahl der periodischen Verformungen zweier jeweils benachbarter Gerüstringe identisch, oder die Anzahl der periodischen Verformungen unterscheidet sich um ein ganzzähliges Vielfaches voneinander. Ist die Anzahl der Verformungen zweier jeweils benachbarter Gerüstringe in den endständigen Bereichen identisch, so kann jede Verformung eines Gerüstringes mit jeder oder beispielsweise mit jeder zweiten, dritten oder vierten korrespondierenden Verformung eines jeweils benachbarten Gerüstringes über die Verbindungsglieder verbunden werden, um das endständige Maschengerüst zu bilden. Dies hat den Vorteil, dass eine Stützprothese mit einer sehr regelmäßigen Maschenstruktur entsteht, die die Innenwand der Aorta an jedem Punkt über die Gesamtlänge des jeweiligen Maschengerüsts hinweg mit gleicher Kraft abstützt. Unterscheidet sich die Anzahl der periodischen Verformungen zweier jeweils benachbarter Gerüstringe um ein ganzzähliges Vielfaches voneinander, so kann jede Verformung eines Gerüstringes mit jeder zweiten, jeder dritten, jeder vierten usw. Verformung eines jeweils benachbarten Gerüstringes in einem jeweiligen endständigen Bereich der Stützprothese über Verbindungsglieder verbunden werden. In dieser Weise kann die Dichte des Maschengerüstes über die gesamte Länge eines jeweiligen endständigen Bereiches hinweg unterschiedlich ausgestaltet werden, um unterschiedlichen klinischen Anforderungen zu genügen, bei denen entlang eines gestützten Abschnittes der Aorta unterschiedliche Stützkräfte erforderlich sind. Dabei ist es möglich, in dieser Art und Weise die Starre der jeweiligen endständigen Maschengerüste unabhängig voneinander einzustellen. Jeder endständige Bereich der Stützprothese gemäß dieser Ausführungsform der Erfindung kann so ausgestaltet sein, dass durch geeignete Auswahl der Anzahl der periodischen Verformungen jeweils benachbarter Gerüstringe die radiale Stützkraft des jeweiligen Maschengerüsts entlang der Gesamtlänge des jeweiligen endständigen Bereichs kontinuierlich ab- oder zunimmt. Auch denkbar gemäß dieser Ausführungsform der Erfindung sind Ausgestaltungen, bei denen die radiale Stützkraft eines jeweiligen Maschengerüstes zur Mitte des Maschengerüsts abnimmt, d. h. diese Stützkraft ist an beiden Enden eines jeweiligen Maschengerüstes größer ist als in der Mitte dieses Maschengerüstes. Umgekehrt sind andere Ausgestaltungen möglich, bei denen die Dichte eines jeweiligen Maschengerüsts durch geeignete Auswahl der Anzahl der periodischen Verformungen jeweils benachbarter Gerüstringe derart gewählt wird, dass die Stützkraft in der Mitte eines jeweiligen endständigen Maschengerüstes am größten ist.

Gemäß einer weiteren Ausführungsform kann die Stützprothese weiterhin zumindest eine Haltevorrichtung enthalten, um nach erfolgter Implantation unerwünschtes Abrutschen innerhalb des Gefässes zu verhindern. Die zumindest eine Haltevorrichtung kann beispielsweise als Haken und Öse, eine Verankerung in der natürlichen Klappen-Ringstruktur (genannt Aortenklappen-Annulus) an der Aortenwand mit Klappfüßchen und/oder Uhrglasverformung eines Endes der Stützprothese ausgestaltet sein.

Gemäß einer weiteren Ausführungsform der vorliegenden Erfindung sind zwei benachbarte Gerüstringe derart phasenversetzt zueinander angeordnet, dass Hochpunkte des einen Gerüstringes mit Tiefpunkten eines jeweils benachbarten Gerüstringes über die Verbindungsglieder verbunden sein können. Bei vielen im Stand der Technik bekannten Gefäßstützprothesen sind zwei benachbarte, periodisch verformten Ringe der Prothesenstruktur über ihre jeweiligen Tief- oder Hochpunkte verbunden, so dass der Hochpunkt eines Ringes mit dem Hochpunkt eines jeweils benachbarten Ringes verbunden oder der Tiefpunkt eines Ringes wird mit dem Tiefpunkt eines jeweils benachbarten Ringes verbunden wird. Werden jedoch zwei benachbarte Gerüstringe punktsymmetrisch um die Stützprothesenlängsachse gegeneinander so rotiert, dass der Hochpunkt eines verformten Gerüstringes dem Tiefpunkt eines jeweils benachbarten verformten Gerüstringes gegenüberliegt, so kann der Hochpunkt des einen Ringes mit dem Tiefpunkt des jeweils benachbarten Ringes über ein Verbindungsglied verbunden werden, wodurch eine sehr hohe Flexibilität der jeweiligen endständigen Maschengerüste erzielt werden kann. Gemäß einer weiteren Ausführungsform ist es möglich, einen Hochpunkt eines Gerüstringes mit einem Mittelpunkt, d. h. mit einem Punkt an oder neben der Mittellinie M wie oben definiert, des jeweils benachbarten Gerüstringes zu verbinden. Gemäß einer weiteren Ausführungsform ist es möglich, einen Mittelpunkt, d. h. einen Punkt an oder neben der Mittellinie M, eines Gerüstringes mit einem Mittelpunkt des jeweils benachbarten Gerüstringes zu verbinden. Gemäß einer weiteren Ausführungsform ist es möglich, einen Tiefpunkt eines Gerüstringes mit einem Mittelpunkt, d. h. mit einem Punkt an oder neben der Mittellinie M, des jeweils benachbarten Gerüstringes zu verbinden.

Vor allem durch eine Hoch-Tief-Verbindung lässt sich die Aortenklappe enthaltende Stützprothese vor dem Einführen ohne weiteres auf den benötigten Durchmesser zusammendrücken, ohne etwas von ihrem vorteilhaften Stützvermögen in den beiden Maschengerüst aufweisenden endständigen Bereichen einzubüßen. In dieser Hinsicht können die Verbindungsglieder zwischen jeweils benachbarten Gerüstringen als Ringe, Klemmen oder sich parallel zur Hauptachse der Stützprothese (auch "Stützprothesenlängsachse" genannt) erstreckende Schlaufen, Fäden, Drähte oder Streben ausgestaltet sein. Hier ist eine Ausgestaltung der Verbindungsglieder als Streben besonders vorteilhaft. In dieser Hinsicht ist es auch vorteilhaft, die lang gezogenen Verbindungsglieder des mittleren Bereichs der Stützprothese als Streben auszugestalten, die mit den zwei dem Mittelbereich benachbarten Gerüstringen verbunden sind.

Die Verbindungsglieder können an den jeweils benachbarten Gerüstringen locker oder starr verbunden sein. Durch ein lockeres Anbringen beispielsweise durch Polymere oder Metalle, die weicher als das Grundmaterial der Stützprothese sind, kann eine sehr hohe Flexibilität in den beiden endständigen, Maschengerüst aufweisenden Bereichen erzielt werden. Eine solche lockere Verbindung der Gerüstringe kann auch durch Draht, einen Clip oder Nahtmaterial bewerkstelligt werden. Es kann auch an den jeweiligen Verbindungsgliedern zumindest eine Sollbruchstelle versehen sein.

Dagegen wirkt sich ein starres Verbinden der Verbindungsglieder an ihren jeweiligen Gerüstringen aussteifend auf das Maschengerüst aus. Bevorzugt sind die Verbindungsglieder, die zwei jeweils benachbarte Gerüstringe verbinden, als starre Streben ausgestaltet, die vorzugsweise in die Struktur der jeweils benachbarten Gerüstringe nahtlos integriert sind. In dem Fall, dass die wachstumsfähige Stützprothese aus einem Röhrenrohling beispielsweise mittels eines Lasers herausgeschnitten werden soll, können solche starre Streben oder Stege auf einfache und dem Fachmann bekannte Weise dadurch geschaffen werden, dass der Rohling an den Stellen, wo später Verbindungsglieder vorgesehen sind, nicht geschnitten wird.

Es ist aber auch denkbar, dass in einem Abschnitt der Stützprothese lockere Verbindungsglieder, dahingegen in einem anderen Abschnitt der Stützprothese starre Verbindungsglieder eingesetzt werden. Auch ist eine Mischung von lockeren und starren Verbindungsgliedern über die gesamte Struktur eines jeweiligen endständigen Maschengerüsts hinweg denkbar. Im Allgemeinen können die jeweiligen endständigen Maschengerüste unabhängig voneinander ausgestaltet sein.

Gemäß einer weiteren Ausführungsform der vorliegenden Erfindung bestehen die Gerüstringe und/oder die Verbindungsglieder zumindest zum Teil aus einem biologisch abbaubaren Material wie beispielsweise aus zumindest einem Metall und/oder zumindest einem Polymer. So kann je nach Bedarf gewährleistet werden, dass mindestens ein Teil der Struktur der Stützprothese sich mit der Zeit auflöst, wobei die TE-Aortenklappe in vorteilhafter Weise mit der Aortenwand zusammenwächst. Hierbei kann die umliegende Struktur der Prothese gemäß der vorliegenden Ausführungsform ganz oder zum Teil aus biologisch abbaubarem Material bestehen. Besteht die Stützprothese ganz aus biologisch abbaubarem Material, ist die Prothesenstruktur nach einiger Zeit ganz aufgelöst, wobei nur die ursprünglich innerhalb der Stützprothese vorliegende Aortenklappe zurückbleibt. Besteht die Stützprothese nur zum Teil aus biologisch abbaubarem Material, verschwindet einige Zeit nach dem Implantieren das biologisch abbaubare Material, wobei Fragmente der Stützprothese, die aus nicht biologisch abbaubarem Material bestehen, zurückbleiben und eine dauerhafte unterstützende Funktion wahrnehmen. Es ist aber auch denkbar, dass die ganze, die Aortenklappe umliegende Struktur der Stützprothese aus nicht biologisch abbaubarem Material besteht, so dass die Aortenklappenprothese als permanente Prothese dient. Die Auswahl der Materialien, d. h. ob die erfindungsgemäße Stützprothese ganz, zum Teil oder überhaupt nicht aus biologisch abbaubarem Material besteht, richtet sich in der Regel nach dem zu behandelnde Patienten. Generell kann aber gesagt werden, dass bei erhöhtem Bedarf an Stützfunktion Ausgestaltungen mit höheren Anteilen an nicht biologisch abbaubaren Materialien eher vorzuziehen sind, denn solche Stützprothesen sind imstande, eine sehr hohe radiale Stützkraft dauerhaft auszuüben.

Gemäß einer weiteren Ausführungsform der vorliegenden Erfindung ist das biologisch abbaubare Material aus zumindest einer Legierung, zumindest einem Polymer oder zumindest einem Edelstahl mit Formgedächtnis ausgewählt. Besonders bevorzugt ist die Legierung mit Formgedächtnis eine Nickel-Titan-Legierung wie beispielsweise das bekannte Nitinol, eine Aluminiumlegierung, eine Magnesiumlegierung oder eine Eisenlegierung. Als Polymere mit Formgedächtnis kommen beispielsweise tert-Butylacrylat oder Poly(ethylenglycol)dimethylacrylat oder PCL kombiniert mit 2,4-Toluoldüsocyanat ethylenglycol in Betracht.

Als biologisch abbaubare Polymere kommen vor allem Polyglykolsäure (PGA), Polymilchsäure (PLA), Polyhydroxyalkanoat (PHA) und Poly-4-hydroxybutyrat (P4HB), Polycaprolactone (PLGA), Polycarbonate, Polyamide, Polyanhydride, Polyaminosäuren, Polyorthoester, Polyacetate, Polycyanoacrylate sowie abbaubare Polyurethane und nicht erodierbare Polymere wie Polyacrylate, Ethylenvinylacetat-Polymere und andere substituierte Zelluloseacetate sowie Derivate davon in Betracht. Polyester werden hierbei bevorzugt. Bevorzugte biologisch abbaubare Polymere umfassen Polymere folgender Gruppen: Polyester der Hydroxycarboxysäuren, Polyanhydride der Dicarboxyester, und Copolymere der Hydroxycarboxysäuren und der Dicarboxyester.

In einer weiteren Ausführungsform besteht das Material aus einem synthetischen Polymer aus mindestens einem der folgenden Monomere: Glykolid, Laktid, p-Dioxanon, Caprolacton, Trimethylencarbonat Butyrolacton. In besonderen Ausführungsformen wird das Material ausgewählt aus einer aus Polymeren oder Copolymeren von Glycolsäure, Milchsäure und Sebacinsäure bestehenden Gruppe. Polyglykolsäurepolymere werden hierbei bevorzugt.

Diese Polymere können sowohl rein als auch in Mischungen aus zwei oder mehreren der genannten Substanzen oder Mischungen dieser Substanzen mit weiteren biologisch abbaubaren Polymeren verwendet werden. In einer bevorzugten Ausführungsform wird ein Mischpolymer aus 80-98% PGA und 20-2% PHA verwendet.

In einer weiteren besonderen Ausführungsform umfasst das biologisch abbaubare Material ein Polyhydroxyalkanoat (PHA). PHA kann dabei mit einem weiteren nichtdegradierbaren Polymer beschichtet werden oder selbst als Beschichtung dienen. Ein bevorzugtes Polyhydroxyalkanoat für diese Verwendung degradiert *in vivo* innerhalb von weniger als 9 Monaten, noch bevorzugter in weniger als 6 Monaten und am stärksten bevorzugt in weniger als 3 Monaten. Eine bevorzugte Zusammensetzung der Polyhydroxyalkanoate beinhaltet 2-, 3-, 4- oder 5-Hydroxysäuren, z. B. Poly-4-hydroxybutyrate. Weiterhin kann die Zusammensetzung ein Poly-4-hydroxybutyrat-co-3-hydroxybutyrat sowie Kombinationen davon beinhalten. Am stärksten bevorzugt wird dabei Poly-4-hydroxybutyrat.

In einer weiteren besonderen Ausführungsform umfasst das biologisch abbaubare Material Homopolymere und Copolymere mit einer beliebigen Kombination folgender Monomere: 3-Hydroxybutyrate, 3-Hydroxyvalerat, 3-Hydroxypropionat, 2-Hydroxybutyrat, 4-Hydroxybutyrat, 4-Hydroxyvalerat, 3-Hydroxyhexanoat, 3-Hydroxyheptanoat, 3-Hydroxyoctanoat, 3-Hydroxynonanoat, 3-Hydroxytridecanoat, 3-Hydroxytetradecanoat, 3-Hydroxypentadecanoat, 3-Hydroxyhexadecanoat, 3-Hydroxyheptadecanoat und 3-Hydroxyoctadecanoat.

Nach einer weiteren Ausführungsform der Erfindung kann die Stützprothese aus einem nicht biologisch abbaubarem Material beschaffen sein und mit einem oder mehreren biologisch abbaubaren Materialien, z.B. Polymeren beschichtet werden. Wiederum ist es nach einer weiteren Ausführungsform der Erfindung auch möglich, dass die Stützprothese aus einem biologisch abbaubaren Material beschaffen, und mit einem oder mehreren nicht biologisch abbaubaren Materialien beschichtet ist. Sowohl das biologisch abbaubare Material als auch das nicht biologisch abbaubare Material können ein Polymer oder ein Metall sein.

Als geeignete nicht oder nur schwer biologisch abbaubare Polymere kommen vor allem Karbon, PTFE, Dacron, PHA und Poly-3-hydroxybutyrat (P3HB) in Betracht.

Gemäß einer weiteren Ausführungsform der vorliegenden Erfindung weisen die Gerüstringe und/oder Verbindungsglieder zumindest eine Sollbruchstelle auf.

Der Begriff "Sollbruchstelle" ist generell so zu verstehen, dass an dieser Stelle im Gerüstring und/oder im Verbindungsglied nicht nur ein Bruch bzw. eine Sprengung bzw. ein Auseinanderbrechen der Sollbruchstelle sondern auch eine Dehnung der Sollbruchstelle möglich ist. Eine Dehnung der Sollbruchstelle ist als eine plastische Deformation dieser aufzufassen, bei der sich die Geometrie der Sollbruchstelle ändert, ohne dass es zu einer Trennung der der Sollbruchstelle benachbarten Teile der Stützprothese kommen muss. Die Dehnung einer Sollbruchstelle kann auch als eine Vorstufe des finalen Bruchs der Sollbruchstelle auftreten. Insoweit schließen sich die Interpretationen einer Sollbruchstelle einerseits als Bruchpunkt, anderseits als Dehnungspunkt nicht gegenseitig aus. Obwohl im nachfolgenden Text überwiegend von Sollbruchstellen im Sinne eines Bruchpunktes gesprochen wird, ist es dem Fachmann somit klar, dass unter Umständen darunter (auch) ein Dehnungspunkt zu verstehen ist.

Das Vorsehen von Sollbruchstellen ermöglicht ein langfristiges Wachstum der Stützprothese, ohne dass die gewünschte Stützfunktion erheblich reduziert wird. So kann die Stützprothese mit Aortenklappe gemäß dieser Ausführungsform vor dem Implantieren in einen Patienten mit homologen, vorzugsweise mit autologen Zellen, vorzugsweise Endothelzellen, Fibroblasten und/oder Myofibroblasten und/oder weitere pluripotente Progenitorzellen und/oder Stammzellen besiedelt werden oder mit bereits *in vitro* hergestelltem Gewebe ergänzt werden. Die so besiedelte Stützprothese wird auf die benötigte Größe bzw. auf den benötigten Durchmesser zusammengedrückt ("gecrimpt", vom englischen "crimp", knautschen) und die so zusammengedrückte Stützprothese wird beispielsweise über einen Venenkatheter an den gewünschten Ort im Körper gebracht. So kann die Stützprothese ihre Stützfunktion im Gefäß nach gegebenenfalls erforderlichem Expandieren mittels eines speziellen zweiteiligen Ballonkatheters wie oben ausgeführt ausüben, ohne dass die empfindliche TE-Aortenklappe beschädigt wird. Wird das Gefäß wie beispielsweise jenes eines Kleinkindes mit der Zeit größer, so kann die Stützprothese mit dem Gefäß mitwachsen. Durch die Aufnahme von vorzugsweise autologen Zellen in die Stützprothese und/oder in die Aortenklappe wird gewährleistet, dass die Wachstumsrate der Stützprothese mit jener des Gefäßes im wesentlichen identisch ist. Mit zunehmendem Lumen des Gefäßes wirken Kräfte auf die Stützprothese, die an den Sollbruchstellen in den beiden endständigen Maschengerüsten zu punktuellen Brüchen führen können, d. h. die endständigen Maschengerüste der Stützprothese können durch die wachstumsbedingten, radial wirkenden Kräfte an ihren Sollbruchstellen auseinander gesprengt werden. So kann sich die Stützprothese zusammen mit der darin angeordnete Herzklappe gemäß dieser Ausführungsform der Erfindung automatisch an einem größer gewordenen Gefäßlumenumfang anpassen, wobei eine hinreichende Stützfunktion noch von dem Zusammenspiel des in der Stützprothese besiedelnden Zellgewebes sowie der noch vorhandenen Fragmente der endständigen Maschengerüste gewährleistet wird.

Es ist möglich, die Sollbruchstellen z.B. in den endständigen Maschengerüsten so auszugestalten, dass sich diese mit der Zeit auflösen, um durch die so entstandenen Schwachstellen eine Sprengung, oder ein Auseinanderbrechen infolge des Wachstumsprozesses zu ermöglichen (passive Sprengung). Auch vorstellbar ist eine aktive Aufsprengung der Sollbruchstellen, beispielsweise mittels Ballon-Angioplastie, um eine bereits implantierte Stützprothese an ein seit dem Implantieren größer gewordenes Lumen anzupassen.

Die zumindest eine Sollbruchstelle kann auch so ausgestaltet sein, dass sie unter äußerer Einwirkung gesprengt, aufgelöst oder geschwächt werden kann. Eine derartige Ausgestaltung der Sollbruchstellen hat den besonderen Vorteil, dass die Sprengung einer Stützprothese, die beispielsweise bereits im frühen Kindesalter implantiert worden ist, später von außen herbeigeführt oder zumindest begünstigt werden kann, ohne dass das mittlerweile gewachsene Kind nochmals operiert werden muss. Die äußere Einwirkung kann aus Schallwellen, zumindest einem magnetischen Feld, einer Kombination aus magnetischem Feld und elektromagnetischem Feld; elektromagnetischer Strahlung; elektrischer Energie und einer beliebigen Kombination davon ausgewählt sein. Die äußere Einwirkung kann von innerhalb oder außerhalb des Körpers erfolgen, sie erfolgt zumindest von außerhalb der Stützprothese. Erfolgt die äußere Einwirkung von innerhalb des Körpers, so kann sie beispielsweise mit Hilfe eines intravaskulären Katheters oder minimal invasiv mit Hilfe der Endoskopie erfolgen. Sie Schallwellen können beispielsweise Ultraschall und/oder Stoßwellen sein. Die Kombination aus magnetischem Feld und elektromagnetischem Feld kann beispielsweise Kernspinresonanz (Magnetic Resonance Imaging, MRI) sein. Die elektromagnetische Strahlung kann beispielsweise Röntgenstrahlung oder Infrarotstrahlung (thermische Energie) sein. Zum Beispiel kann durch eine lokale Temperaturerhöhung ein steiler Abfall der Steifheit einer Sollbruchstelle (Dehnung der Sollbruchstelle durch plastische Deformation) oder sogar ein Bruch der Sollbruchstelle erreicht werden.

Unabhängig davon, ob die Stützprothese aktiv oder passiv gesprengt wird, ermöglichen die Sollbruchstellen der erfindungsgemäßen Stützprothese, dass die Größe, z. B. der Durchmesser der erfindungsgemäßen Stützprothese an den Durchmesser des Gefäßes angepasst wird, ohne dass die Segel der TE-Aortenklappe beschädigt werden.

Die konkrete Ausgestaltung der Sollbruchstellen ist nicht beschränkt, solange sie ihre Funktion wie obenstehend beschrieben ausüben können. Konkret lässt sich diese Funktion auf unterschiedliche Art und Weise realisieren. Bei einer Stützprothese aus einem metallischen Material kann eine Sollbruchstelle beispielsweise als zumindest eine Perforation in einer Gerüstringstrebe oder im Verbindungsglied ausgestaltet sein. Solche Perforationen können unterschiedlicher Art sein, beispielsweise als eines oder mehrere durchgehende Löcher und/oder als eine oder mehrere nicht durchgehende Vertiefungen im Stützprothesenmaterial. Die Form der Löcher bzw. der Vertiefungen ist nicht eingeschränkt: sie kann rund, elliptisch, polygonal, rechteckig und/oder quadratisch sein. Die Vertiefung kann auch als eine Kerbe im Material der Stützprothese ausgestaltet sein, die zum Beispiel quer oder schräg über die Breite eines Gerüstringes und/oder eines Verbindungsgliedes verläuft. Es ist auch möglich, solche Kerben, oder Verengungen auf beiden Seiten einer Strebe eines Gerüstringes und/oder Verbindungsgliedes so anzuordnen, dass zwei Kerben dieselbe Sollbruchstelle von beiden Seiten des Gerüstrings bzw. des Verbindungsglieds (d. h. von oben und unten) bilden, d. h. sowohl von den der Gefäßwand als auch des Gefäßlumens zugewandten Seiten der Stützprothese. Alternativ oder auch ergänzend dazu können an derselben Stelle, d. h. in derselben Höhe der Strebe wie die zwei oben und unten angeordneten Kerben, eine oder zwei Kerben rechts und links angeordnet sein. So kann die Stärke einer Sollbruchstelle dahingehend eingestellt werden, dass man sie aus einer, zwei, drei oder vier Kerben, die alle in derselben Höhe der Strebe oder des Verbindungsglieds angeordnet sind, schafft. Obwohl diese Ausführungsform eine Strebe und/oder ein Verbindungsglied mit vierkantigem Querschnitt annimmt, ist anzumerken, dass Entsprechendes bei Streben und/oder Verbindungsgliedern mit anders ausgestalteten Querschnitten gilt.

Je nach den konkreten physiologischen Anforderungen ist es auch denkbar, dass zumindest eine Sollbruchstelle in zumindest einem langgezogenen Verbindungsglied geschaffen wird, um das Wachstum entlang der Stützprothesenlängsachse zu ermöglichen oder begünstigen.

Es ist auch möglich, eine Sollbruchstelle als eine punktuelle oder linienförmige Überschreitung der plastischen Verformbarkeit einer Strebe des Gerüstringes auszugestalten, und/oder punktuelle oder linienförmige Unterschiede in der Strukturdichte des Stützprothesematerials zu schaffen. Sollbruchstellen können auch durch das Schaffen punktueller oder linienförmiger Schädigungen in der Oberflächenstruktur oder der inneren Struktur des Gerüstringes und/oder des Verbindungsgliedes (beispielsweise durch Erhöhung der stellenweisen Porosität des Materials, beispielsweise durch die Aufnahme von Nanopartikeln im Bereich der Sollbruchstelle) bewerkstelligt werden. Auch vorstellbar ist eine örtlich begrenzte oder unbegrenzte Mischung des Grundmaterials der Stützprothese mit Polymer und/oder mit Polymerinterponaten, Drähten, Fäden und/oder organischen Interponaten wie beispielsweise Kollagen.

Eine Sollbruchstelle kann auch als Schnitt oder Spalt im Material der Stützprothese (d.h. im Gerüstring oder im Verbindungsglied) ausgestaltet sein. Dieser Schnitt oder Spalt kann mit einem nicht metallischen Material, beispielsweise einem biologisch abbaubaren Material im Bereich der Sollbruchstelle überdeckt sein, so dass zunächst nur das biologisch abbaubare Material die Sollbruchstelle zusammenhält. Dies kann beispielsweise so erreicht werden, dass das biologisch abbaubare Material beidseitig des Schnitts oder Spalts auf dem Material des Gerüstringes oder des Verbindungsgliedes überlappt und mit diesem verbunden ist. Löst sich das biologisch abbaubare Material mit der Zeit im Körper auf, so reduziert sich sein Haltvermögen entsprechend, bis entweder die infolge des Wachstums entstehenden Sprengkräfte das Haltvermögen des biologisch abbaubaren Materials übersteigen oder sich das biologisch abbaubare Material ganz auflöst und den darunterliegenden Schnitt oder Spalt freilegt, so dass die Stützprothese ungehindert wachsen (oder mittels Ballon-Angioplastie aufgedehnt werden) kann. Durch geeignete Auswahl des an der Sollbruchstelle verwendeten, biologisch abbaubaren Materials kann die Geschwindigkeit des Wachstumsprozesses gesteuert werden, denn es ist bekannt, dass unterschiedliche biologisch abbaubare Materialien sich unterschiedlich schnell *in vivo* auflösen.

Wie oben erwähnt, kann die zumindest eine Sollbruchstelle auch so ausgestaltet sein, dass sie dass sie unter äußerer Einwirkung gesprengt, aufgelöst oder geschwächt werden kann.

Da die Menge des biologisch abbaubaren Materials an den Sollbruchstellen in der Regel sehr gering ist, sind bei dessen Auflösung keine Entzündungsreaktionen zu befürchten, wie im Stand der Technik beim Auflösen von Stützprothesen, die ganz aus biologisch abbaubarem Material bestehen, der Fall ist.

Eine entsprechende Ausgestaltung unter Verwendung eines biologisch abbaubaren Materials ist auch dann möglich, wenn statt eines Schnitts oder eines Spalts und wie oben beschrieben eine Perforation und/oder eine Kerbe im Material der Stützprothese vorliegen.

Es ist auch möglich, unabhängig von der Ausgestaltung als Perforation, Kerbe, Vertiefung, Spalt oder Schnitt an der zumindest einen Sollbruchstelle mehrere Schichten unterschiedlicher biologisch abbaubarer Materialien anzubringen, so dass eine zeitlich gestaffelte Aufsprengung der Stützprothese ermöglicht wird. So kann beispielsweise gewährleistet werden, dass die Auflösung der Sollbruchstelle anfangs sehr langsam verläuft und erst nach langer Zeit (d. h. nachdem die langsam sich auflösende aüßere Schicht verschwunden ist) die Auflösungsrate der Sollbruchstelle und das damit verbundene Wachstumspotential der Stützprothese zunimmt, oder umgekehrt.

Es ist auch möglich, dass eine Stützprothese unterschiedlich ausgestaltete Sollbruchstellen aufweist. Zum Beispiel kann eine Stützprothese eine oder mehrere Sollbruchstellen als Perforationen ohne biologisch abbaubares Material und eine oder mehrere Sollbruchstellen als mit einem oder mehreren biologisch abbaubaren Materialien überdeckte Schnitte und/oder Spalte aufweisen. So kann gewährleistet werden, dass unterschiedliche Bereiche derselben Stützprothese innerhalb derselben Zeit *in vivo* unterschiedlich schnell wachsen. Die erfindungsgemäße Stützprothese erlaubt daher einen sehr hohen Grad an Flexibilität bei der Anpassung an die unterschiedlichsten physiologischen Anforderungen.

Es ist auch möglich, im Bereich der Sollbruchstellen nicht biologisch abbaubares Polymer zu verwenden. Die Sollbruchstelle kann, wie oben beschrieben, ausgestaltet sein, d. h. als Perforation, Kerbe, Vertiefung, Spalt oder Schnitt. Wird beispielsweise beidseitig überlappend über einen Schnitt oder einen Spalt im Gerüstring und/oder im Verbindungsglied nicht biologisch abbaubares Polymer gebracht, so hängt die Stärke der Sollbruchstelle und damit auch das Wachstumsvermögen der Stützprothese von der Stärke des nicht biologisch abbaubaren Polymers ab. Bei bekannter Materialstärke des nicht biologisch abbaubaren Polymers kann in dieser Weise die Stärke bzw. die Wachstumseigenschaften der Stützprothese je nach physiologischen Anforderungen gesteuert werden.

Es ist auch möglich, zumindest eine Sollbruchstelle wie oben ausgeführt in oder an zumindest einem der langgezogenen Verbindungsglieder des Mittelbereiches sowie an zumindest einen Verbindungspunkt zumindest eines langgezogenen Verbindungsgliedes mit dem ihm zugewandten Gerüstring vorzusehen.

Gemäß einer weiteren Ausführungsform der vorliegenden Erfindung ist die zumindest eine Sollbruchstelle bevorzugt an oder neben dem Mittelpunkt zwischen einem Hoch- und Tiefpunkt des periodisch verformten Gerüstringes zumindest eines endständigen Bereiches, d. h. an oder neben dem Schnittpunkt des Gerüstringes mit der, wie oben definierten Mittellinie M, angeordnet. Die zumindest eine Sollbruchstelle kann aber auch zusätzlich oder alternativ dazu an den Hoch- und/oder Tiefpunkten der periodischen Verformungen des Gerüstringes angeordnet sein. Beim Wachsen oder Sprengen der Stützprothese in der oben dargestellten Weise werden radial von innen nach außen strebende Kräfte auf die beiden endständigen Maschengerüste der Stützprothese ausgeübt. Wird die Stützprothese während des Wachstums- oder Angioplastievorgangs aufgedehnt, so konzentrieren sich diese Kräfte an den Hoch- und Tiefpunkten der Gerüstringe, da diese Hoch- und Tiefpunkte der endständigen Maschengerüste der Stützprothese einen großen Teil ihres Flexionsvermögens verleihen. Werden die Sollbruchstellen ausschließlich an den Hoch- und Tiefpunkten des periodisch verformbaren Gerüstringes angeordnet, besteht die Gefahr, dass das gesamte Maschengerüst frühzeitig gesprengt wird, wodurch unter Umständen einen Großteil seiner Stützfunktion in unerwünschter Weise verlorenginge. Werden die Sollbruchstellen aber ausschließlich oder vorwiegend an oder neben dem Mittelpunkt zwischen den Hoch- und Tiefpunkten des periodisch verformbaren Gerüstringes angeordnet, kann unter Umständen das Auseinanderbrechen des Maschengerüsts verzögert werden.

Durch das gezielte Mischen der Orte, an denen Sollbruchstellen angeordnet sind, kann man die Sprengneigung der Stützprothese in sehr vorteilhafter Weise steuern. Je mehr Sollbruchstellen an Hoch- und/oder Tiefpunkten eines periodisch verformbaren Gerüstringes angeordnet sind, desto größer ist die Sprengneigung des jeweiligen endständigen Maschengerüsts. Je mehr Sollbruchstellen an oder neben den Mittelpunkten eines periodisch verformbaren Gerüstringes angeordnet sind, desto geringer ist die Sprengneigung des jeweiligen endständigen Maschengerüsts.

Die Sollbruchstellen weisen nach einer bevorzugten Ausführungsform ein nicht metallisches Material, beispielsweise ein Polymermaterial auf oder können auch aus diesem bestehen. Dieses Material kann ein Polymer sein, und kann biologisch abbaubar oder nicht biologisch abbaubar sein. Eine solche Ausgestaltung bringt einige Vorteile mit sich.

Erstens besitzen Polymermaterialien in der Regel eine geringere Reißfestigkeit als metallische Materialien, wobei die Reißfestigkeit direkt vom Degradationsgrad des verwendeten Materials abhängt. Bei der wachstumsfähigen Stützprothese mit Aortenklappe gemäß der vorliegenden Ausführungsform wirkte sich eine erhöhte Reißfestigkeit an den Sollbruchstellen wachstumshemmend aus, da die Sollbruchstellen nur schwer auseinandergerissen werden könnten. Umgekehrt kann durch die Auswahl eines Materials mit erhöhter Reißtendenz an den Sollbruchstellen die Wachstumsfähigkeit der Stützprothese begünstigt werden. So hat man die Möglichkeit, durch Verwendung eines nicht metallischen Materials einer bekannten Reißfestigkeit an den Sollbruchstellen, Stützprothesen mit unterschiedlichen Wachstumsfähigkeiten bereitzustellen. So kann die Wachstumsfähigkeit der bereitgestellten Stützprothese den spezifischen klinischen Anforderungen eines jeden Patienten angepasst werden.

Zweitens wird durch die Ausgestaltung der Sollbruchstellen mit nicht metallischem Material gewährleistet, dass nach dem Aufbrechen der endständigen Maschengerüste infolge des Wachstums oder des Expandierens keine scharfkantigen Ecken aus Metall gebildet werden, die später die abgestützte Gefäßwand verletzen könnten, oder die abbrechen und einen entfernten Ort im Gefäßsystem beeinträchtigen könnten.

Für den Fall, dass das Maschengerüst der Stützprothese aus einem metallischem Material und die Sollbruchstellen aus einem nicht metallischen Material bestehen oder dieses umfassen, wird bevorzugt das nicht metallische Material in dem Bereich der Sollbruchstellen durch Beschichten auf das metallische Material der Stützprothese aufgebracht. Dabei weist das metallische Material des Maschengerüsts zumindest in dem Bereich der Sollbruchstellen, d. h. unterhalb der Beschichtung aus nicht metallischem Material, bevorzugt eine polierte Oberflächenstruktur bzw. eine abgerundete Form auf. So wird gewährleistet, dass auch nach dem Aufbrechen der Sollbruchstellen und gegebenenfalls nach dem Auflösen des nicht metallischen Materials eine glatte Oberfläche der Maschengerüste bzw. keine scharfe Kanten an diesen Sollbruchstellen zurückbleiben, wodurch der erwähnte Vorteil des verminderten Verletzungsrisikos realisiert wird. Verfahren zum Polieren von metallischen Feinstrukturen wie beispielsweise das Elektropolieren sind dem Fachmann bekannt.

Nach einer weiteren Ausführungsform der Erfindung kann die Sollbruchstelle so ausgestaltet sein, dass ihre Rigidität oder Haltvermögen erst ab einer Beanspruchung von vorbestimmter Dauer bzw. von vorbestimmter Stärke nachlässt. Beispielsweise kann die Sollbruchstelle einer für den Aortenbereich gedachten wachstumsfähigen Stützprothese so beschaffen sein, dass der Schwellenwert der maximal tolerierten, durch das Pumpen des Herzens verursachten mechanischen Beanspruchung des Sollbruchstellenmaterials erst ab einer bestimmter Anzahl von Herzschlägen erreicht wird. Ausgehend von einer durchschnittlichen Herzschlagrate kann man so effektiv die Dauer vorprogrammieren, nach der die Stützprothese nach der Implantation anfängt, sich aufzulösen oder sich zu dehnen. Dies ermöglicht eine Steuerung der Stützprothesenwachstumsrate, was insbesondere bei dem Einsatz im Kleinkind vor Vorteil ist, da dadurch die Anzahl an chirurgischen Eingriffen wesentlich reduziert wird. Es ist natürlich auch vorstellbar, dass die Beanspruchung, der die Sollbruchstelle ausgesetzt werden soll, einer anderen Natur als mechanisch ist.

Gemäß einer weiteren Ausführungsform der vorliegenden Erfindung beträgt die Länge der lang gezogenen Verbindungsglieder das 0,75 bis 3-fache der Amplitude *A.* Bevorzugt beträgt die Länge der lang gezogenen Verbindungsglieder im Mittelbereich der Stützprothese das 1 bis 2-fache der Amplitude A. Durch Einstellen der Länge der lang gezogenen Verbindungsglieder im Mittelbereich der Stützprothese kann auf vorteilhafte Weise an unterschiedliche anatomische Erfordernisse angepasst werden, d. h., man kann je nach den anatomischen Erfordernissen in dem Bereich, in dem die Stützprothese eingebracht werden soll, den Mittelbereich der Stützprothese verkürzen oder verlängern.

Die TE-Aortenklappe kann vorteilhafterweise an den Mittelbereich der Stützprothese genäht, geklebt, geklemmt oder gewebt sein, wobei es bevorzugt ist, die TE-Aortenklappe an den Mittelbereich der Stützprothese zu nähen. Im idealen Fall wird die TE-Aortenklappe derart in einer Höhe des Mittelbereichs befestigt, dass sie nach dem Implantieren der erfindungsgemäßen Stützprothese an ihrem nativen Ort liegt, d. h. an dem Ort, wo früher die körpereigene Aortenklappe gelegen hat. Bevorzugt liegt die Aortenklappe in der distalen Hälfte des Mittelbereichs der Stützprothese, d. h. in der Hälfte der Stützprothese, die sich im implantierten Zustand näher am Herz befindet.

Die folgenden Abbildungen und Beispiele erläutern in nicht einschränkender Weise die Erfindung. Die Abbildungen zeigen:

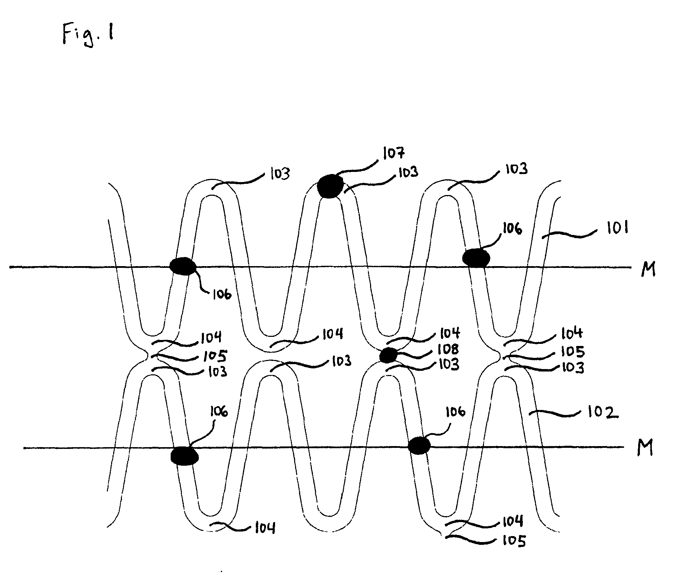

- Fig. 1:: Eine Draufsicht eines Abschnittes des Maschengerüstes in einem endständigen Bereich einer Stützprothese gemäß einer Ausführungsform der Erfindung. Dargestellt werden ein erster periodisch verformter Gerüstring (101) und ein zweiter periodisch verformter Gerüstring (102), jeweils mit Hochpunkten (103) und Tiefpunkten (104), die in deren Mitte die Umfangsmittellinie M definieren. Der erste und zweite Gerüstring (101 und 102) werden über als Streben ausgestaltete Verbindungsglieder (105) miteinander verbunden. Hier weist das dargestellte Maschengerüst Sollbruchstellen auf. Diese werden als schwarze Punkte dargestellt, die in der gezeigten Ausführungsform an oder neben der Mittellinie M (Sollbruchstellen 106), an einem Hochpunkt (Sollbruchstelle 107) sowie in einem Verbindungsglied (Sollbruchstelle 108) angeordnet sind.
- Fig. 2A:: Einen Abschnitt (201) eines Gerüstringes oder Verbindungsglieds mit einer Sollbruchstelle, die als Perforation (202) ausgestaltet ist.
- Fig. 2B:: Einen Abschnitt (201) eines Gerüstringes oder Verbindungsglieds mit einer Sollbruchstelle, die mit oben und unten angeordneten, dreieckförmign Kerben (203) ausgestaltet ist.
- Fig. 2C:: Einen Abschnitt (201) eines Gerüstringes oder Verbindungsglieds mit einer Sollbruchstelle, die mit oben angeordneter dreieckförmigen Kerbe (204) ausgestaltet ist.
- Fig. 2D:: Einen Abschnitt (201) eines Gerüstringes oder Verbindungsglieds mit einer Sollbruchstelle, die mit oben und unten angeordneten viereckförmigen Kerben (205) ausgestaltet ist.
- Fig. 2E:: Einen Abschnitt (201) eines Gerüstringes oder Verbindungsglieds mit einer Sollbruchstelle, die mit abgerundetem Schnitt ausgestaltet ist, und die mit einem Polymer (207) beschichtet ist.
- Fig. 2F:: Einen Abschnitt (201) eines Gerüstringes oder Verbindungsglieds mit einer Sollbruchstelle, die mit Stufenschnitt (208) ausgestaltet ist, und die mit einem Polymer (207) beschichtet ist.
- Fig. 2G:: Einen Querschnitt (211) eines Gerüstringes oder Verbindungsglieds mit einer Sollbruchstelle, die mit oben und unten angeordneten viereckförmigen Kerben (205) ausgestaltet ist.

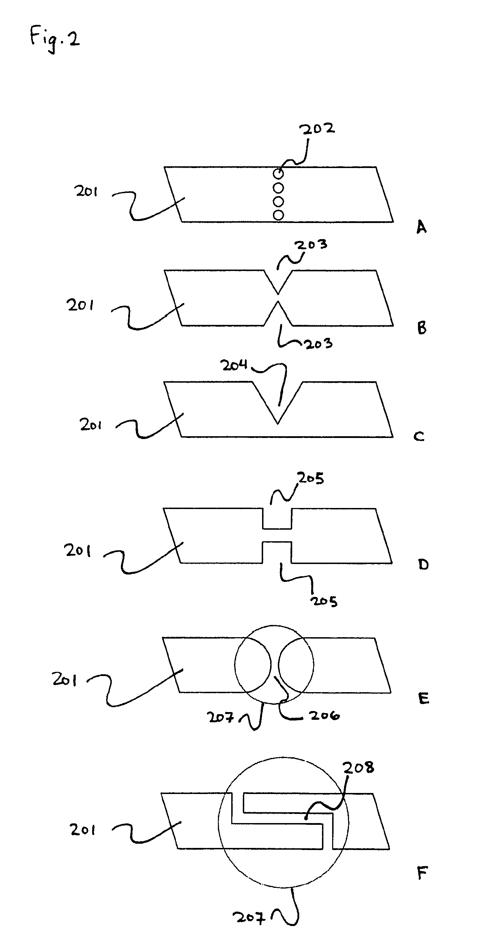

- Fig. 2F:: Einen Querschnitt (211) eines Gerüstringes oder Verbindungsglieds mit einer Sollbruchstelle, die mit oben, unten, links und rechts angeordneten viereckförmigen Kerben (205) ausgestaltet ist.

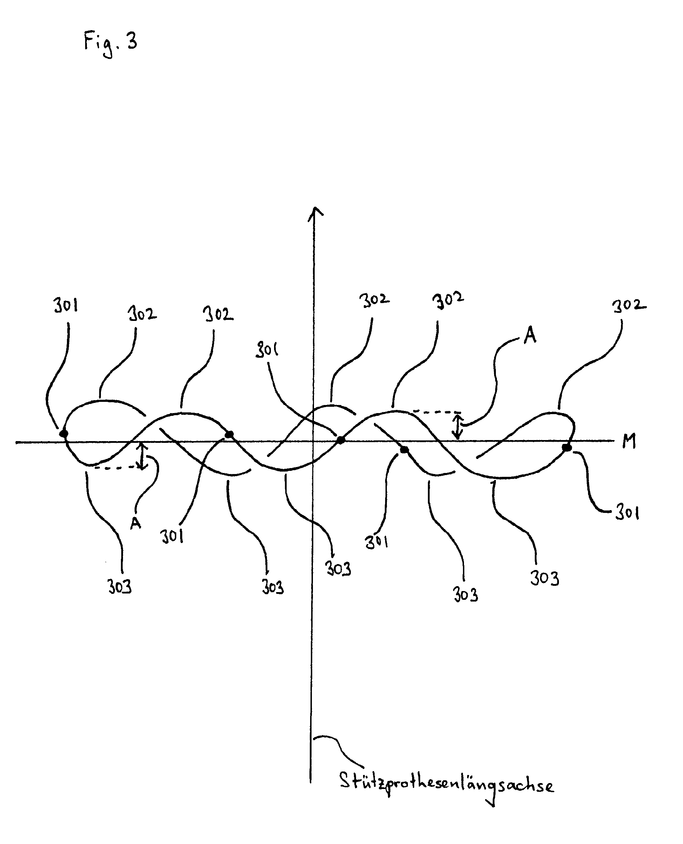

- Fig. 3: Eine Perspektivansicht eines Gerüstringes im endständigen Bereich mit sinusförmigen Verformungen. Der gezeigte Gerüstring weist 5 Hochpunkte (302), 5 Tiefpunkte (303) und 5 Sollbruchstellen (301) an oder neben der senkrecht zur Stützprothesenlängsachse verlaufenden Mittellinie M auf. Ebenfalls gezeigt ist die Amplitude A. Nicht gezeigt sind Verbindungsglieder, die der dargestellte Gerüstring mit einem benachbarten Gerüstring im Maschengerüst verbinden. Auch nicht gezeigt sind die langgezogenen Verbindungsglieder.

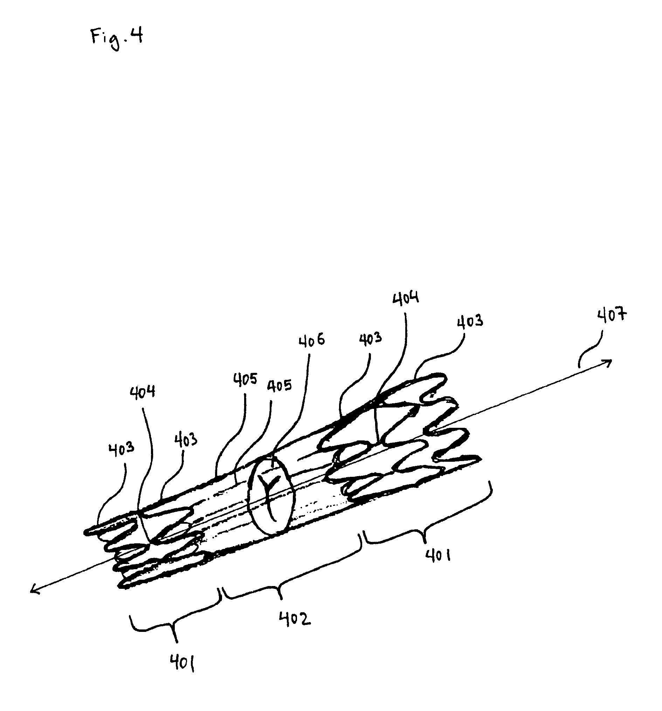

- Fig. 4: Ein Perspektivansicht einer TE-Herzklappe tragenden rohrförmigen Stützprothese nach einer Ausführungsform der Erfindung. Die gezeigte Stützprothese weist zwei auf die Stützprothesenlängsachse (407) bezogene, endständige Bereiche (401), einen Mittelbereich (402), langgezogene Verbindungsglieder (405) sowie eine TE-Aortenklappe (406) auf. Gerüstringe (403) in den jeweiligen endständigen Bereichen (401) werden über Verbindungsglieder (404) miteinander verbunden.

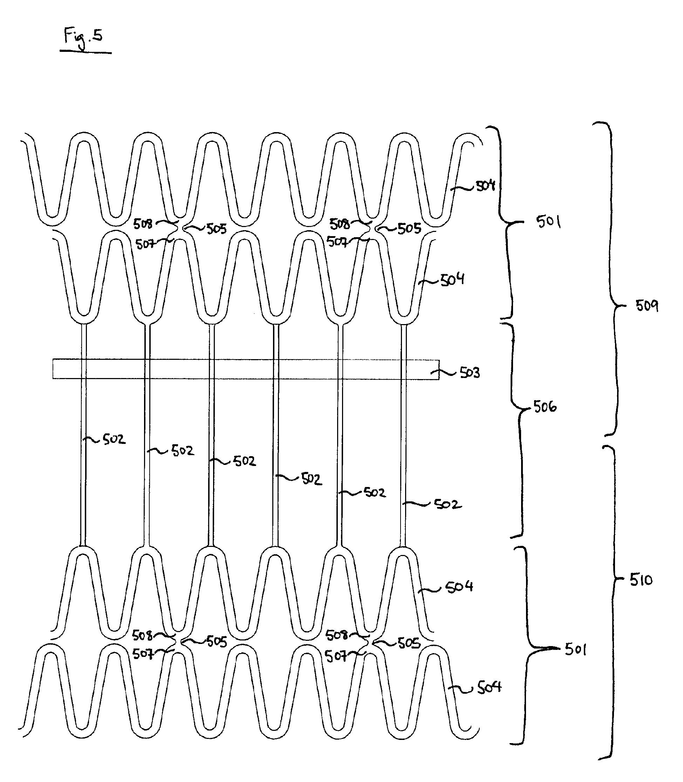

- Fig. 5: Eine Draufsicht eines Abschnitts einer Stützprothese gemäß einer Ausführungsform der Erfindung. Die gezeigte Stützprothese weist zwei auf die Stützprothesenlängsachse bezogene, endständige Bereiche (501) und einen dazwischenliegenden Mittelbereich (506) auf. Die jeweiligen endständigen Bereiche sind über mehrere langgezogene Verbindungsglieder (502) miteinander verbunden. Die zwei Gerüstringe (504) in jedem der zwei endständigen Bereiche sind über Verbindungsglieder (505) an deren jeweiligen Hochpunkten (507) und Tiefpunkten (508) miteinander verbunden. Hier sind die Verbindungsglieder (505) als Stege ausgestaltet. Die TE-Aortenklappe (503) ist in Seitenansicht dargestellt. Die gesamte Stützprothese kann in zwei Hälften entlang der Stützprothesenhauptachse unterteilt werden: in einer distalen Hälfte (509), die im implantierten Zustand näher zum Herz liegt, und in einer proximalen Hälfte (510), die im implantierten Zustand vom Herz weiter entfernt liegt. In der gezeigten Ausführungsform befindet sich die TE-Aortenklappe (503) in der distalen Hälfte (509) der Stützprothese bzw. des Mittelbereichs (596).

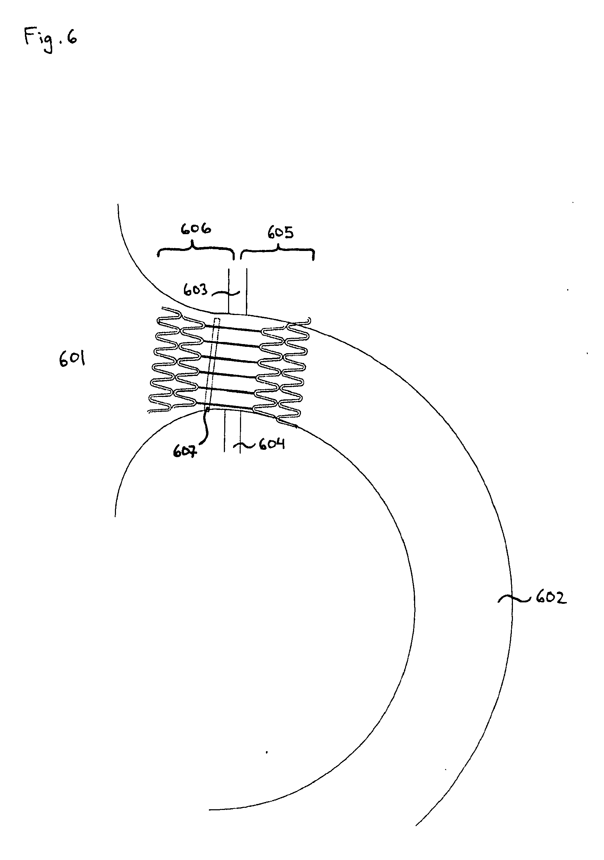

- Fig. 6: Eine schematische Ansicht einer in Fig. 5 dargestellten Stützprothese, wie sie im Aortenbereich nach erfolgter Implantation liegen kann. Die Stützprothese liegt zwischen dem Herz (601) und der Aorta (602) an ungefähr der Stelle, wo sich normalerweise die Aortenklappe befinden würde. Die linken und rechten Koronargefäße (603 bzw. 604 oder 604 bzw. 603, je nach dem ob die dargestellte Ansicht von oben oder von unten ist) sind ebenfalls dargestellt. Die übrigen Teile der Stützprothese sind wie oben in Fig. 5 dargestellt und erläutert. Hervorgehoben wird hier lediglich die TE-Aortenklappe (607) in Seitenansicht sowie die proximalen (605) und distalen (606) Hälften der Stützprothese. Wie ersichtlich ist, werden die Koronargefäße (603, 604) durch den lockeren Mittelbereich der Stützprothese mit seinen langgezogenen Verbindungsgliedern nicht verlegt bzw. okkludiert. Auch die sich in der distalen Hälfte (606) der Stützprothese befindende TE-Aortenklappe (607) beeinträchtigt den Blutfluß durch die Koronargefäße (603, 604) nicht.

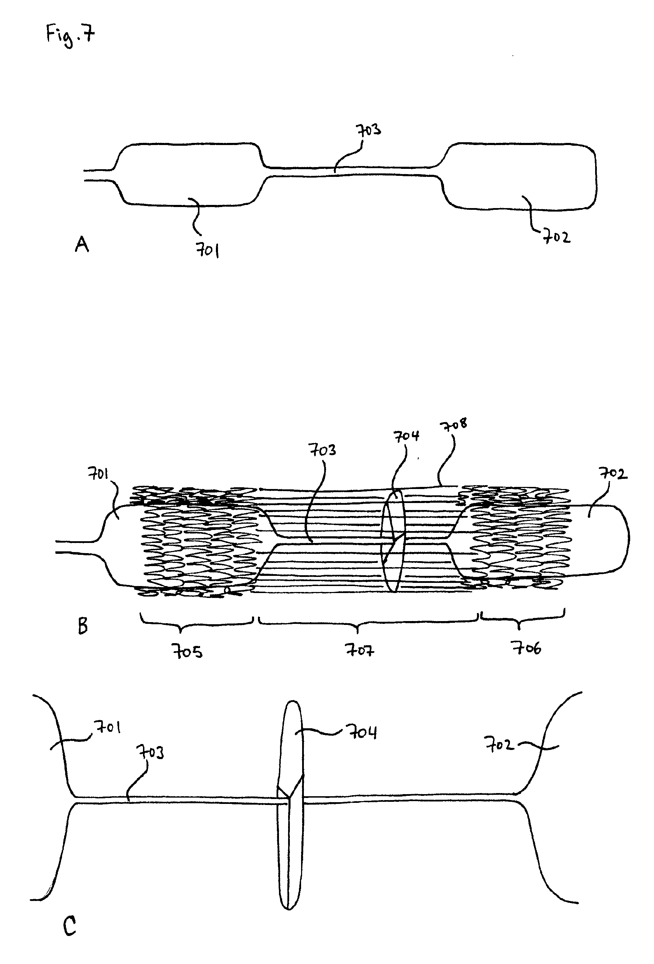

- Fig. 7A: Einen speziellen zum Expandieren einer erfindungsgemäßen Stützprothese geeigneten Angioplastie-Ballon. Der Ballon wird im vollexpandierten Zustand dargestellt. Der Ballon besteht im Wesentlichen aus drei Bereichen: einem proximalen expandierbaren Bereich (701); einem mittleren nicht oder nur gerinfügig expandierbaren Bereich (703); und einem distalen expandierbaren Bereich (702).
- Fig. 7B: Eine Perspektivansicht des speziellen Angioplastie-Ballons aus Fig. 7A. Dargestellt ist die Verwendung des speziellen Ballons zum Expandieren einer Ausführungsform der erfindungsgemäßen Stützprothese. Zunächst wird der spezielle, noch nicht expandierte Ballon in einer Stützprothese eingeführt, so dass die Höhen des proximalen expandierbaren Bereichs (701), des mittleren Bereichs (703) bzw. des distalen expandierbaren Bereichs (702) etwa den korrespondierenden Höhen des endständigen Bereichs (705), des mittleren Bereiches (707) inklusiv der TE-Aortenklappe (704) bzw. des endständigen Bereiches (706) der Stützprothese entsprechenden. Dabei ist darauf zu achten, dass sich die Aortenklappe (704) in der Höhe des mittleren Bereichs (703) des Ballons befindet. Wird der so eingeführte Ballon dann expandiert, werden die ein Maschengerüst aufweisenden, endständigen Bereiche (705, 706) der Stützprothese expandiert, nicht jedoch der mittlere Bereich (707), wo die Aortenklappe (704) an den langgezogenen Verbindungsgliedern (708) befestigt angeordnet ist. So wird vermieden, dass beim Expandieren des Ballons die empfindlichen Segel der TE-Aortenklappe beschädigt werden.
- Fig. 7C: Eine Detailansicht der Interaktion zwischen dem mittleren Bereich (703) des Ballons und der TE-Aortenklappe (704). Der Ballon wird im vollexpandierten Zustand dargestellt, wie an den proximalen (701) sowie distalen (702) expandierbaren Bereichen ersichtlich ist. Nicht dargestellt werden die übrigen Teile der die TE-Aortenklappe enthaltenden Stützprothese. Wie aus der Zeichnung ersichtlich ist, bleibt der mittlere Ballonbereich (703) auch in vollexpandiertem Zustand immer noch sehr dünn, so dass er durch den die Mitte der Aortenklappe hindurchtreten kann, ohne die umgebenden Klappensegel zu beschädigen.

Das nachfolgende Beispiel erläutert in nicht einschränkender Weise, wie eine wachstumsfähige Stützprothese hergestellt werden kann.

### Beispiel 1: Fertigung einer klappentragenden Stützprothese

Im Allgemeinen umfasst die Produktion einer wachstumsfähigen Stützprothese folgende Verfahrensschritte:
1. Laserschneiden
2. Elektropolieren
3. Kontrollieren/Reinigen
4. Verbindung Klappe - Stützprothese

### 1.1. Laserschneiden

Die Stützprothese wird aus hochpräzisem Material (z. B. 316L, L605 oder Nitinol) unter Verwendung eines Festkörperlasers gefertigt. Um das Design der Stützprothese frei gestalten zu können, wird ein Verfahren angestrebt, das eine beliebige Schnittführung auf einem Rohr ermöglicht. Ein Laser erfüllt in Verbindung mit einem Koordinatentisch, einer Rundachse und einer speziell angefertigten Rohrführung, die das Rohr präzise in den erforderlichen Freiheitsgraden unter dem Laserschneidkopf führt, diese Anforderungen. Dabei werden die Stützprothesenstruktur und die in diesem Fall mechanischen Sollbruchstellen (wie beispielsweise in Fig. 2 dargestellt) in einem Arbeitsgang in das Rohr geschnitten. Der Mittelteil der Stützprothese ist dabei so gestaltet, dass die langgezogenen Streben die Klappe während der Implantation nicht verletzen können. Nach der Implantation müssen die Streben die Klappe sicher offen halten und dürfen die Koronargefäße nicht verlegen (siehe Fig. 6). Nach Entfernen der "Fenster", d.h. der ausgeschnittenen Bereiche bleiben die Streben der Stützprothese und damit die fertige Stützprothesenstruktur zurück. Dazu gehören sowohl die Gerüstringe als auch die diese verbindenden Verbindungsglieder.

### 1.2. Elektropolieren

Durch das dem Fachmann allgemein bekannte Elektropolieren werden die durch das Laserschneiden entstandenen Stützprothesenkanten zur Minimierung der Gefäß- und Implantationsballonverletzung abgerundet und die Stützprothesenoberflächen geglättet. Zum Einsatz kommt ein elektrochemisches Polierverfahren, das an die speziellen Gegebenheiten der Stützprothesenherstellung angepasst ist. Die Anpassung für die Stützprothesenbearbeitung umfasst hauptsächlich eine Poliervorrichtung, die einen gleichmäßigen Stromfluss während des Poliervorgangs gewährleistet. Dabei werden die Stützprothesen im Elektropolierbad kontinuierlich bewegt und gleichzeitig unter Spannung gesetzt.

### 1.3. Kontrollieren/Reinigen

Nach der Politur durch das oben beschriebene Elektropolierverfahren werden die Stützprothesen durch Dekapieren und mehrere Spülungen von Politurrückständen gereinigt. Anschließend werden sie optisch kontrolliert sowie vermessen und verpackt. Bei der optischen Kontrolle wird die gesamte Oberfläche beurteilt. Dabei werden Stützprothesen aussortiert, die u.a. folgende Kriterien nicht erfüllen:
- gleichmäßige, glatte Oberfläche
- durchgängige Kantenverrundung ohne Spitzen

### 1.4. Verbindung Klappe - Stützprothese

TE-Herzklappen können nach bekannten Verfahren vor allem unter Anwendung homologer oder autologer menschlicher Zellen hergestellt werden (siehe z. B. EP 1499366). Die so hergestellte TE-Klappe muß beschädigungsfrei mit der Stützprothese verbunden werden. Die gewählte Verbindungsart muss dergestalt mit den Verbindungsstreben der Stützprothese zusammenarbeiten, dass ein sicheres Zusammenfalten (Crimpen) der Stützprothese sowie eine leckagefreie Implantation der Klappe gewährleistet ist. Zur Auswahl stehen mechanische Befestigungsarten wie nähen, klemmen, kleben oder binden, bei denen die einzelnen Verbindungsstreben mit der Klappe verbunden werden. Alternativ kann eine dezellularisierte TE-Herzklappe oder ein noch nicht zellularisiertes Polymergerüst für eine Herzklappe zuerst in eine Stützprothese integriert bzw. befestigt werden und erst dann nach bekannten Verfahren mit homologen oder autologen Zellen besiedelt werden.

## Patentansprüche

1. Rohrförmige Stützprothese aufweisend zwei auf die Stützprothesenlängsachse bezogene endständige Bereiche und einen zwischen den beiden endständigen Bereichen angeordneten Mittelbereich, wobei jeder endständige Bereich ein Maschengerüst aus zumindest zwei miteinander über Verbindungsglieder verbundenen, punktsymmetrisch um die Stützprothesenlängsachse angeordneten Gerüstringen aufweist, wobei der Mittelbereich durch langgezogene Verbindungsglieder gebildet ist, die mit den jeweils dem Mittelpunkt der Stützprothesenlängsachse am nächsten angeordneten Gerüstringen verbunden sind, und wobei eine mittels Tissue-Engineering hergestellte Aortenklappe im Mittelbereich befestigt/und oder integriert ist.

2. Stützprothese gemäß Anspruch 1, wobei zumindest ein Gerüstring des Maschengerüsts *n* periodische, längs der Stützprothesenlängsachse sich erstreckende, Hoch- und Tiefpunkte bildende Verformungen aufweist, die eine sich auf die Stützprothesenlängsachse bezogene Amplitude A aufweisen, wobei n = 16-70, bevorzugt 20-56, besonders bevorzugt 24-42.

3. Stützprothese nach Anspruch 2, wobei die Verformungen sinusförmig, rechteckig, sägezahnförmig oder dreieckig ausgestaltet sind.

4. Stützprothese nach Anspruch 2 oder 3, wobei die Anzahl der periodischen Verformungen zweier jeweils benachbarter Gerüstringe identisch ist oder sich um ein ganzzähliges Vielfaches voneinander unterscheidet.

5. Stützprothese nach einem der Ansprüche 2 bis 4, wobei zwei benachbarte Gerüstringe derart phasenversetzt zueinander angeordnet sind, dass Hochpunkte eines Gerüstrings mit Tiefpunkten eines jeweils benachbarten Gerüstrings über die Verbindungsglieder verbunden sind.

6. Stützprothese nach einem der Ansprüche 1-5, wobei die Verbindungsglieder als Ringe, Klemmen oder sich parallel zur Stützprothesenlängsachse erstreckende Schlaufen, Fäden, Drähte oder Streben ausgestaltet sind.

7. Stützprothese nach einem der Ansprüche 1-6, wobei die Gerüstringe und/oder die Verbindungsglieder zumindest zum Teil aus einem biologisch abbaubaren Material bestehen.

8. Stützprothese nach Anspruch 7, wobei das biologisch abbaubare Material aus zumindest einer Legierung, zumindest einem Polymer oder zumindest einem Edelstahl mit Formgedächtnis ausgewählt ist.

9. Stützprothese nach Anspruch 8, wobei die Legierung mit Formgedächtnis eine Nickel-Titan-Legierung, eine Aluminum-Legierung, eine Magnesium-Legierung oder eine EisenLegierung ist.

10. Stützprothese nach Anspruch 7, wobei das biologisch abbaubare Polymer Polyglykolsäure (PGA), Polymilchsäure (PLA), Polyhydroxyalkanoat (PHA), Poly-4-Hydroxybutyrat (P4HB), Polycaprolactone (PLGA), Polycarbonate, Polyamide, Polyanhydride, Polyaminosäuren, Polyorthoester, Polyacetate, Polycyanoacrylate sowie abbaubare Polyurethane und nicht erodierbare Polymere wie Polyacrylate, Ethylenvinylacetat-Polymere, andere substituierte Zelluloseacetate sowie Derivate davon, Polyester der Hydroxycarboxysäuren, Polyanhydride der Dicarboxyester, Copolymere der Hydroxycarboxysäuren und der Dicarboxyester, ein synthetisches Polymer aus mindestens einem Glykolid, Laktid, p-Dioxanon, Caprolacton, Trimethylencarbonat und/oder Butyrolacton, Polymere oder Copolymere von Glykolsäure, Milchsäure und Sebacinsäure, Polyhydroxyalkanoat-Zusammensetzungen von 2-, 3-, 4- oder 5-Hydroxysäuren, z. B. Poly-4-hydroxybutyrate, ein Poly-4-hydroxybutyrat-co-3-hydroxybutyrat, Homopolymere und Copolymere mit einer beliebigen Kombination von 3-Hydroxybutyrate, 3-Hydroxyvalerat, 3-Hydroxypropionat, 2-Hydroxybutyrat, 4-Hydroxybutyrat, 4-Hydroxyvalerat, 3-Hydroxyhexanoat, 3-Hydroxyheptanoat, 3-Hydroxyoctanoat, 3-Hydroxynonanoat, 3-Hydroxytridecanoat, 3-Hydroxytetradecanoat, 3-Hydroxypentadecanoat, 3-Hydroxyhexadecanoat, 3-Hydroxyheptadecanoat und 3-Hydroxyoctadecanoat oder eine Kombination davon ist.

11. Stützprothese nach einem der Ansprüche 1-10, wobei die Gerüstringe und/oder Verbindungsglieder zumindest eine Sollbruchstelle aufweisen.

12. Stützprothese nach Anspruch 11, wobei die zumindest eine Sollbruchstelle an oder neben dem Mittelpunkt zwischen einem Hoch- und Tiefpunkt des periodisch verformten Gerüstringes angeordnet ist.

13. Stützprothese nach Anspruch 11 oder 12, wobei die zumindest eine Sollbruchstelle aus einem nicht metallischen Material bestehen.

14. Stützprothese nach einem der Ansprüche 2-13, wobei die Länge der langgezogenen Verbindungsglieder das 0,75-3-fache, bevorzugt das 1-2-fache der Amplitude A beträgt.

15. Stützprothese nach einem der Ansprüche 12-14, wobei die zumindest eine Sollbruchstelle derart ausgestaltet ist, dass sie unter äußerer Einwirkung gesprengt, aufgelöst oder geschwächt werden kann.

16. Stützprothese nach Anspruch 15, wobei die äußere Einwirkung ausgewählt ist aus: Schallwellen; zumindest einem magnetischen Feld; einer Kombination aus magnetischem Feld und elektromagnetischem Feld; elektromagnetischer Strahlung; elektrischer Energie und einer beliebigen Kombination davon.

17. Stützprothese nach Anspruch 16, wobei die Schallwellen Ultraschall und/oder Stoßwellen sind.

18. Stützprothese nach Anspruch 16, wobei die Kombination aus magnetischem Feld und elektromagnetischem Feld Kernspinresonanz (Magnetic Resonanz Imaging, MRI) ist.

19. Stützprothese nach Anspruch 16, wobei die elektromagnetische Strahlung Röntgenstrahlung oder Infrarotstrahlung (thermische Energie) ist.

20. Stützprothese nach einem der Ansprüche 15-19, wobei die äußere Einwirkung von innerhalb oder außerhalb des Körpers erfolgt.

21. Stützprothese nach Anspruch 20, wobei die Einwirkung von innerhalb des Körpers mit Hilfe eines intravaskulären Katheters oder minimal invasiv mit Hilfe der Endoskopie erfolgt.

22. Stützprothese nach Anspruch, wobei das biologisch abbaubare Polymer mit Formgedächtnis aus tert-Butylacrylat, Poly(ethylenglycol)dimethylacrylat oder PCL kombiniert mit 2,4-Toluoldiisocyanat ethylenglycol ausgewählt ist.
